(19)

Europäisches
Patentamt
European
Patent Office
Office européen
des brevets

(11) **EP 1 831 387 B1**

(12) **EUROPEAN PATENT SPECIFICATION**

(45) Date of publication and mention
of the grant of the patent:
**19.05.2010 Bulletin 2010/20**

(21) Application number: **05856192.9**

(22) Date of filing: **22.12.2005**

(51) Int Cl.:
***C12P 19/04*** *(2006.01)*

(86) International application number:
**PCT/BR2005/000259**

(87) International publication number:
**WO 2006/066377 (29.06.2006 Gazette 2006/26)**

(54) **CONTINUOUS FERMENTATION PROCESS TO PRODUCE BACTERIAL CELLULOSIC SHEETS**

KONTINUIERLICHES FERMENTATIONSVERFAHREN ZUR HERSTELLUNG VON BAKTERIELLEN CELLULOSEFILMEN

PROCÉDÉ DE FERMENTATION EN CONTINU POUR PRODUIRE DES FEUILLES CELLULOSIQUES BACTÉRIENNES

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.12.2004 BR PI0405990**

(43) Date of publication of application:
**12.09.2007 Bulletin 2007/37**

(73) Proprietor: **Bionext Produtos Biotecnológicos Ltda.**
**São Paulo SP (BR)**

(72) Inventors:
• **FARAH, Luiz Fernando Xavier**
**Jardim das Américas, Curitiba - PR (BR)**
• **CORAL, Lucila Adriani**
**Bairro Gloria, Joinville - SC (BR)**
• **ARCHANJO, Cristiane Do Rocio**
**Curitiba - PR (BR)**
• **PODLECH, Pablo Angel Sanchez**
**Bairro Rebouças, Curitiba - PR (BR)**

(74) Representative: **Petruzziello, Aldo**
**Racheli & C. S.p.A.**
**Viale San Michele del Carso, 4**
**20144 Milano (IT)**

(56) References cited:
**EP-A1- 0 831 101     WO-A-86/02095**
**WO-A1-2004/050986     FR-A1- 2 615 864**
**JP-A- 9 094 094     JP-A- 62 265 990**
**US-A- 5 114 849**

• **JONAS ET AL: "Production and application of microbial cellulose" POLYMER DEGRADATION AND STABILITY, vol. 59, 1998, pages 101-106, XP025505631**
• **RECOUVREUX ET AL: "Evidências da presença de celulose no biofilme formado por Chromobacterium violaceum" August 2005 (2005-08), pages 1-6, XP002531194 Retrieved from the Internet: URL:www.intelab.ufsc.br: 8080/port/public/S INAFERM2005-Recouvreux%20et%20al%2002.pdfw ww.intelab.ufsc.br:8080/port/public/SINAFE RM2005-Recouvreux%20et%20al%2002.pdf> [retrieved on 2009-06-08]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention refers to a continuous industrial production process for bacterial cellulosic sheets with high purity and specific chemical and physical properties. The bacterial celluloses sheet can be used for various purposes.

Any of the culture media mentioned in the literature (e. g. Biochemical Journal, Vol. 58, pp. 345-352, 1956; Brazilian Journal of Chemical Engineering, vol. 13, pp. 47-50, 1996; Carbohydrate Polymers, vol. 40, pp. 137-143, 1999; Journal of Bioscience and Bioengineering, vol. 88, pp. 183-188, 1999; Biotechnology and Bioengineering, vol.68, pp. 345-372, 2000; Biotechnology and Applied Biochemistry, vol. 35, pp. 125-132, 2002) may be used for the continuous process, object of the present invention. Obviously, working conditions will depend on the fermentation media and microorganisms used. The numeric results, presented below just as examples, were obtained from a strain selected from *Acetobacter xylinum* and a fermentation medium containing glucose (10 to 50 g/l), yeast extract (0.5 to 4.0 g/l), monopotassium phosphate (0 to 2.0 g/l), heptahydrated magnesium sulphate (0 to 1.5 g/l) and ethyl alcohol (0.5 to 2.0% by volume). The experiments were made under the temperature of $30 \pm 2$ °C on 50 cm x 30 cm x 12 cm trays (interface area between the fermentation medium and air, 1500 cm$^2$).

The first references to cellulose produced by bacteriae go back to Brown, A.J. (1886). J.Chem.Soc. 49, 432, Tarr and Hibbert (1931). Can. J. Res. 4,373 , Khouvine, Y., Bull. Soc. Chem., 18, 1325 (1936) and Hestrin, Aschener and Mager in 1947 published studies on fermentation media aiming at cellulose production (Hestrin, S. Aschner, M. & Mager, J. (1947), Synthesis of cellulose by resting cells of Acetobacter xylinum. Nature. London 159, 64). Khausal and Walker published in 1951 a study also presenting different fermentation media for cellulose production (Kaushall, R. Walker, T.K. & Drummond, D. G. (1951), Observations on the formation and structure of bacterial cellulose. Biochem. J. 50, 128).

In 1977, Clovin published a study describing the effects of the addition of glucose to the fermentation medium for cellulose production (Colvin, J. R. Chènè, L., Sowden, L. C. & Takai, M. (1977), Purification and properties of a soluble polymer of glucose from cultures of Acetobacter xylinum. Canadium Journal of Biochemistry 55, 1057-1063). Clovin also mentioned the formation of a sheet composed of cellulose fibrils that become visible after 6 to 8 hours of medium inoculation. In a publication of 1980 (Colvin, J. R. (1980). The biosynthesis of cellulose. In Biochemistry of Plants, vol. 3, Carbohydrates; Structure and Function, pp. 543-570, edited by J. Preiss. New York: Academic Press), Clovin reported the extrusion of cellulose by the bacteria membrane, stating that the microfibrils spontaneously assemble in the culture medium to form a cellulose fibril. Lepard et al, in a publication of 1975, reported that growing microfibrils are linearly extended polyglucosane structures, in principle highly hydrated and up to 100 nm wide (Leppard, G.G. Sowden, L.C. & Colvin J. R. (1975) Science 189, 1094-1095).

Later on, still in suspension on the liquid medium, they gradually associate to form a consolidated fibril. According to the literature and observations made, such cellulose fibrils randomly associate, structuring a sheet that floats on the fermentation medium.

For decades, bacterial cellulose has been produced in Far Eastern countries (e.g. the Philippines and Thailand) for the production of "coconut cream" (a sweet obtained by cooking cellulosic sheet in sugar syrup). In the process used, the medium is water or coconut milk and the fermentation lasts weeks. After fermentation, the sheet is harvested and the fermentation medium is replaced by a new medium to begin a new cycle. Furthermore, since it is a manual process, there is no control of the fermentation temperature and environment air humidity, thus resulting in a product with variable quality. As a result, it is difficult to implement an economically viable industrial operation where the quality of the product can be assured, due to the lack of regularity in the conditions and period of fermentation of the raw material.

Patent BR PI 9204232 discloses environmental conditions to obtain cellulosic sheets, with the requirement of air renewal at a flow of 5 m$^3$/h for a sheet surface equal to 1 m$^2$. Such technology presents the inconveniences of high operational cost (e.g. frequent change of absolute air filters) and the need of strict and complex controls for various parameters (e.g. control of air humidity) generating high production costs. Furthermore, it mentions the formation of a lamella adhered to the lower face of the sheet, with different characteristics from the sheet and which must be removed.

Patents BR 8404937 of 1984 and US 4,912,049 of 1990 describe a fermentation medium to produce sheets by *Acetobacter xylinum,* where the source of nitrogen is an extract of *Tea sinensis* and the source of carbon is sucrose. It is currently known that *Acetobacter xylinum* uses glucose as a carbon source. The use of sucrose by said patent slows down the reproduction of the bacteria, increasing the time of fermentation to obtain the sheet, since the hydrolysation of sucrose into glucose and fructose is required.

Borzani and Souza (in a paper published in Biotechnology Letters, vol. 17, pp. 1271-1272, 1995) demonstrated that the cellulosic sheet is formed in its interface with air and not in its interface with the medium.

Patent application BR PI 0205499-0 (WO 2004/050986) describes the production of bacterial cellulose from a given fermentation medium in covered trays. In this case, the process comprises:

    1. preparation of the fermentation medium;
    2. inoculation of the medium;

3. filling of the fermentation trays;

4. fermentation;

5. collection of the sheets formed; and

6. change of used fermentation trays for clean ones.

This process is discontinuous and presents considerable "dead times" (items 3, 5 and 6) between one batch and another. The following case for example: a) 200 trays are being used for each batch; b) the fermentation time to obtain fine pellicles of a given grammage (weight of a paper sheet in grams per square meter) is 48 hours; c) two people carry out all the operations for each batch. The first "dead time" of about three hours in the example presented here is spent with the distribution of trays in the chamber, that provides appropriate conditions of temperature and air circulation and their filling with inoculated fermentation medium (1.5 liters per tray). After 48 hours of fermentation, the formed sheets will be harvested and the trays with residual media will be taken from the chamber. This is the second "dead time" which, in this example, is also of about three hours. A full batch will therefore take 54 hours, from which six (12.5% of the fermentation time) are "non productive hours". Furthermore, about 300 liters of inoculated medium should be prepared and 200 trays should be washed and prepared for another batch every 48 hours.

Bearing in mind said inconveniences and aiming to fulfill a gap existing on the market, the present invention was developed. The present invention refers to a continuous production process for sheets, minimizing dead times, reducing labor and increasing productivity of the process. By the process of the present invention, multiple bacterial cellulose sheets are obtained with no need to replace a new fermentation medium in the tray.

Experiments for the production of cellulosic sheets by bacteriae made on trays and in industrial scale have shown that, if the volume and the composition of the fermentation medium placed in the tray follow given requirements, the tray will be able to produce several sheets, all of them with practically the same content of cellulose, without replacement of the nutrients consumed by the bacteriae (see Example 1).

The present invention will be better understood by looking at the attached figures, given as mere examples, but not limiting:

- Figure 1 - schematic representation of the continuous process with the production of (M) sheets per tray without re-feeding of the tray with non inoculated new medium;
- Figure 2 - medium volume reduction graph (D) in the tray when a sheet is harvested whose formation time is TF;
- Figure 3 - productivity increase graph ($P_C/P_D$) regarding the ratio between "dead time"/fermentation time (R) and the total number of sheets that may be produced in a tray (N).

The continuous process for the production of cellulosic sheets, object of the present invention, comprises the following steps:

a) preparation of the fermentation medium; dissolution of medium components in water;

b) sterilization of the medium: heating of the medium to 115°C, keeping it at this temperature for 20 minutes;

c) cooling of the medium until its temperature reaches 30°C;

d) medium inoculation with a suspension of bacteriae.

e) distribution of the medium in the trays;

f) fermentation: the trays are left to rest (the fermentation time depends on the grammage of the final pellicle desired);

g) harvest of the sheets, draining them over the corresponding trays;

h) repetition of the fermentation in the trays left to rest during fermentation and harvest of the sheets from the trays, draining them over the corresponding trays until, after collecting and draining the sheet, the volume of medium in the tray is between 15% and 25% of the initial volume;

i) when the volume of 15% to 25% of the initial volume is reached, a new fermentation medium should be added to the tray (prepared according to the initial fermentation medium, i.e. dissolution of medium components in water; medium sterilization; heating of the medium to 115°C, keeping it at that temperature for 20 minutes; cooling the medium until its temperature reaches 30°C) in order to obtain the initial volume again; and

j) repetition of the fermentation in the trays left to rest during fermentation and collecting the sheets from the trays, draining them over the corresponding trays until, after draining the sheet, the volume of medium in the tray is between 15% and 25% of the initial volume when new fermentation medium is added and the cycle is re-started.

As in any continuous process, the sequence of cycles described in item "j" is not indefinitively extended. Interruptions in the process may be caused by various reasons, among them we can mention: 1) equipment maintenance and repair; 2) formation of filaments in suspension on the medium, frequently formed during fermentation, and whose accumulation may even damage the formation of sheets; 3) accidental contamination of the medium by harmful microorganisms.

The main advantages of the continuous process, object of this invention, over the discontinuous process, may be observed in Example 2,which compares the results of experiment made in the conditions indicated in Example 1, and the results obtained in the production of sheets by a discontinuous process.

The additional information required to understand the following steps of the continuous process are also described here, as follows: sheet draining, preparation of inoculum, fermentation time and volume of medium in the tray and its variation.

1. Sheet draining

The cellulose in the sheet taken from the trays, which will form the final pellicle, represents less than 20% of the mass of the sheet. In a sheet of approximately 600 g, for instance, there is only 3 to 4 g of cellulose. The rest of the sheet is medium impregnated. On the other hand, said water medium that impregnates the sheet is much more concentrated in bacteriae (5 to 10 times more) than the free medium in the tray. For these reasons, it is important to recover part of this medium, both to prepare the inoculum and for the continuous process, object of this invention.

Sheet draining is the operation to recover part of the water medium that permeates the sheet.

As an example, Table 1 shows the approximate volume of water medium recovered by draining the sheet.

Table 1

| Mass of the sheet(g) | Volume of drained medium (ml) |
|---|---|
| 100 to 200 | 200 to 300 |
| 300 to 400 | 400 to 600 |
| 600 to 700 | 400 to 600 |
| 900 to 1100 | 400 to 600 |

Preparation of the inoculum

Two cases should be considered as follows:

1) preparation of the inoculum from a pure culture of bacteriae or lyophilized bacteriae;
2) preparation of the inoculum from the residual water medium existing in the tray after the fermentation is finished.

In any of the cases mentioned, the volumes of medium used, temperature and incubation time depend on the composition of the medium and the microorganism used. Considering that there are many cultivation media described in the literature, and numerous cellulose producing bacteriae known, it is not possible to propose one single detailed method to prepare the inoculum applicable to all cases.

The methods described in summary below, presented here just as mere examples, have achieved good results in many cases. However, we should stress that, no matter the method used, it is essential to take due care to avoid contamination of the medium by microorganisms present in the work environment.

The preparation of the inoculum (first case) from a pure culture of bacteriae or lyophilized bacteriae comprises the following steps:

a) inoculation of a container with culture medium with bacteriae from a pure culture or lyophilized ones;
b) incubation for 48 hours;
c) stirring of the sheet formed and the rest of the medium using a glass rod (said stirring substitutes in part the draining of the small sheet formed);
d) inoculation of four Erlenmeyer flasks containing 100 to 120 ml of the fermentation medium each, with 10 to 15 ml portions of the water medium (rich in bacteriae) obtained from item c,
e) incubation of the four flasks for 48 hours;
f) stirring of the contents of the four flasks with a glass rod;
g) inoculation of a tray containing 1200 to 1300 ml of the fermentation medium with the water medium of the four flasks;
h) incubation of the tray for 48 hours;
i) taking the sheet formed, draining it over the residual medium left in the tray;
j) the water medium obtained in the previous item will serve as inoculum for other trays (the volume of inoculum should be equal to 10%-20% of the volume of fermentation medium in the trays to inoculate).
k) repetition of the tray incubation cycle for 48 hours; taking the sheet formed, draining it over the residual medium present in the tray; using the water medium obtained as inoculum until a sufficient volume of water medium (10%-20% of the volume of fermentation medium in the trays to inoculate) rich in bacteriae to inoculate the volume of fermentation medium to be used for industrial production is obtained.

In the second case, as long as due care is taken for industrial production, the preparation of the inoculum for a new batch of fermentation medium involves simply the operation mentioned in item "i" (taking the sheet formed, draining it over the residual medium existing in the tray) with the appropriate number of production trays.

Fermentation time

The fermentation time to form a tray that, when submitted to final treatments, produces a cellulose pellicle of a given grammage, depends on the composition of the fermentation medium, the bacteria strain and the temperature.
Experiments made with the fermentation medium and the bacteria already cited, and at the temperature of 30°C $\pm$ 1°C, reached the results presented in Table 2.

Table 2

| G (g/m$^2$) | TF (hours) |
| --- | --- |
| 6 to 8 | 24 |
| 17 to 20 | 48 |
| 30 to 33 | 72 |
| 52 to 56 | 120 |
| 210 to 230 | 456 |
| G = grammage of the final cellulose pellicle TF = fermentation time to form the sheet | |

Considering the average values of grammages for each fermentation time, i. e.: G = 7.2 g/m$^2$ (for TF = 24 hours), G = 19.0 g/m$^2$ (for TF = 48 hours), G = 31.4 g/m$^2$ (for TF = 72 hours), G = 55.0 g/m$^2$ (for TF = 120 hours), G = 220 g/m$^2$ (for TF = 456 hours), the approximate value of grammage for different fermentation time values can be calculated with the following equation:

$$G = -4.4 + 0.4922.TF$$

Volume of medium in the tray and its variation

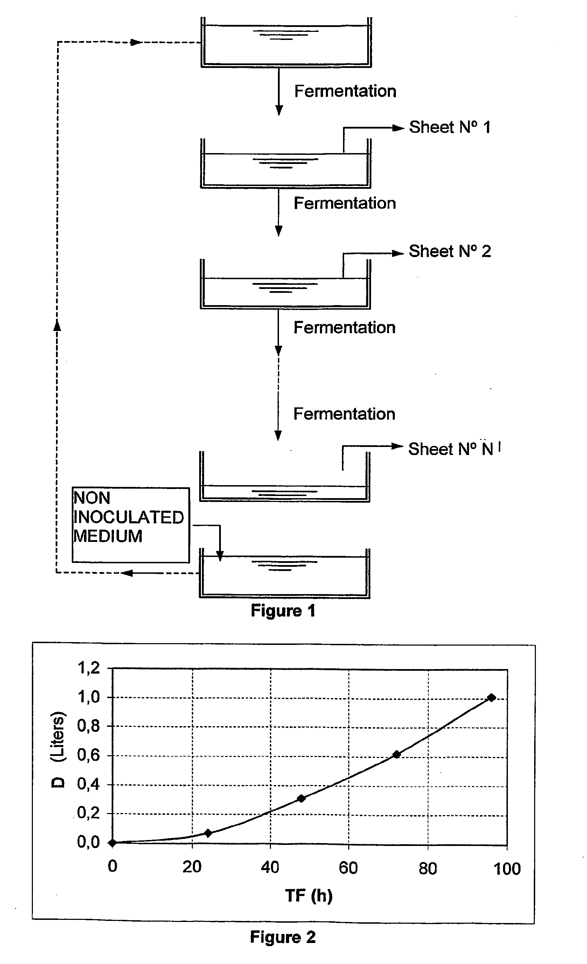

In the continuous process, object of this invention, the volume of medium in the tray is reduced with time for two reasons: 1) loss by evaporation; 2) loss by the harvest of the sheet formed, even draining it.
Loss by evaporation mainly depends on the temperature, the humidity of the air that circulates in the chamber storing the trays, speed of said air over the trays and the interface area between the sheet being formed and the air. The speed of the evaporation loss is usually expressed in milliliters of medium per day and per square meter of the area of the sheet/air interface. Increase in temperature, reduction of air humidity and increase in air speed in the chamber cause increase in the speed of evaporation loss, thus the control of said parameters to reach a practically constant and relatively low value of said speed is of utmost importance.
Speeds of evaporation loss of 250 ml/(m$^2$.day) and 2,200 ml/(m$^2$.day) were found in industrial units, which shows the impossibility of adopting an average value for the calculation.
The reduction of the volume of the medium in the tray as a consequence of the harvest of a sheet formed there will be proportional to the mass of the sheet. The bigger the sheet, the bigger will be the reduction. On the other hand, the mass of the formed sheet will be proportional to the area of the sheet/air interface and the required time of fermentation to form a sheet.
Experiments made with those already mentioned fermentation medium and bacteria in trays have shown that the reduction of the volume of medium in the tray (D) as a consequence of the harvest of a sheet corresponding to time for fermentation TF varies as shown in Table 3 and Figure 2.

Table 3

| TF (hours) | D (liters) |
|---|---|
| 24 | 0,070 |
| 48 | 0,310 |
| 72 | 0,620 |
| 96 | 1,010 |

The equation that correlates D and TF, represented by the curve of Figure 2 is:

$$D = 0.000160.(TF)^{1.93}$$

If the interface area were A (in cm$^2$), the equation would be:

$$D = \frac{A}{1500}.0.000160.(TF)^{1.93}$$

Example 3 shows how to calculate the volume of inoculated medium to be put in a tray, for the continuous process, object of this invention, to enable harvest of a given number of sheets corresponding to a given fermentation time, with no need to replace, in the tray, new fermentation medium (see Figure 1).

**Example 1:** From a tray containing 10 liters of fermentation medium, it was possible to obtain in 28 days 14 sheets (48 hours of fermentation per sheet) which, after being submitted to final treatments, supplied dry cellulose pellicles with *gramatura* of 18 g/m$^2$, and the consumption of the medium components by the producing bacteriae did not damage the characteristics of the final product.

**Example 2:** Two hundred trays are used to produce sheets, whose fermentation time is 48 hours, by the continuous process (see Example 1) and by the discontinuous process. The results are as follows:

Continuous process

a) each tray produces 14 sheets in 28 days, with no need to replace the medium in the trays reaching a total of 2,800 sheets produced;
b) the initial volume of inoculated medium in each tray is 10 liters;
c) the total initial volume of inoculated medium to be prepared is 2,000 liters;
d) there is only one "dead time" of three hours to prepare the trays;
e) the total fermentation time to produce 14 sheets per tray is 672 hours (14 x 48 hours);
f) there is only one "dead time" of three hours to unload and take off the trays;
g) the total production time of 2800 sheets is 678 hours (3 + 672 + 3);
h) process productivity is 2800/678 = 4.13 sheets per hour (or 99.1 sheets per day).

2. Discontinuous process

a) each tray produces one sheet in 48 hours, which is then unloaded and substituted by another tray;
b) the initial volume of inoculated medium in each tray is 1,5 liters;
c) the volume of inoculated medium to be prepared for the production of 200 sheets (one per tray) is 300 liters and, for the production of 2800 sheets, is 4200 liters. In this case, there are 14 operations to prepare 300 liters of inoculated medium;
d) there are 14 "dead times" of three hours each to prepare the trays, thus resulting in a total of 42 hours (14 x 3);
e) the total fermentation time is also 672 hours (14 x 48);

f) there are 14 "dead times" of three hours each to unload and take off the trays, thus resulting in a total of 42 hours (14 x 3);

g) the total production time of 2800 sheets is 756 hours (42 + 672 + 42);

h) process productivity is 2,800/756 = 3.70 sheets per hour (or 88.9 sheets per day).

Table 4 summarizes the values presented in Example 2. We should highlight two important points in Example 2: the number of sheets produced per tray in the continuous process, without unloading trays and substituting them for others, is surely higher than 14, which increases the advantages of the continuous process over the discontinuous one; 2) The volume and polluting load of effluents in the continuous fermentation process are lower than in the discontinuous process.

Table 4

|  | Discontinuous process | Continuous process |
|---|---|---|
| Total volume of inoculated medium (liters) | 4.200 | 2.000 |
| Number of operations to prepare the inoculated medium | 14 | 1 |
| Number of loading operations of trays | 14 | 1 |
| Number of unloading operations of trays | 14 | 1 |
| Total "dead" time to load trays (hours) | 42 | 3 |
| Total "dead" time to unload trays (hours) | 42 | 3 |
| Total time of fermentation (hours) | 672 | 672 |
| Total time of production (hours) | 756 | 678 |
| Number of draining operations = number of produced sheets | 2.800 | 2.800 |
|  |  |  |
|  |  |  |
|  |  |  |
| Productivity (sheets/hour) | 3,70 | 4,13 |
| Productivity (sheets/day) | 88,9 | 99,1 |

**Example 3:** From one tray, we intend to harvest ten drained sheets corresponding to a fermentation time of 72 hours, without replacing consumed nutrients. The speed of losses by evaporation is 500 ml/(m$^2$.day). After harvesting the tenth sheet, the residual volume of the medium in the tray should be equal to 15% of the initial volume. Calculate the volume of inoculated medium to be put in the tray.

a) volume of medium taken from the tray with the ten sheets:

$$10 \times 0.62 = 6.2 \text{ liters}$$

b) volume of medium lost by evaporation:

$$0.15 \ (m^2) \times 500 \ mL/(m^2.day) \times 30 \ (days) = 2250 \ mL = 2.25 \ liters$$

c) being V the initial volume of inoculated medium, the residual volume after harvest the ten sheets should be of 0.15 V;
d) the value of V will then be:

$$V = 6.2 + 2.25 + 0.15V$$

$$V = 9.94 \text{ liters} \approx 10 \text{ liters}$$

The continuous process, object of this invention, presents, in summary, the following advantages over the discontinuous process:

- reduction of labor;
- increase in productivity;
- reduction of the volume of fermentation effluents;
- reduction of the polluting load of fermentation effluents.

Important remark: the numerical values presented are valid for the fermentation medium and bacteria strain used in the experiments. However, this does not invalidate the above mentioned conclusions.

We should present the deduction of the equation to calculate how many times the productivity of the continuous process, object of this invention, is higher than the discontinuous process.

$(TF) =$   Fermentation time to form the sheet

$(TM) =$   "dead time" spent in tray loading and unloading operation

$R = (TM)/(TF) =$   Ratio between "dead time" and fermentation time

$N =$   number of sheets that may be produced in a single tray, by the continuous process without the need to unload the tray and substitute it for another one

In the continuous production process of N sheets, there will be only one loading operation, one unloading operation and N successive fermentations. If we indicate by $T_C$ the total time to produce N sheets, we have:

$$T_C = (TM) + N*(TF)$$

But as $(TM) = (R*(TF))$, the result is:

$$T_C = (TF) \times (R+N)$$

Thus, the productivity of the continuous process $(P_C)$ will be:

$$P_C = \frac{N}{T_C} = \frac{N}{(TF) \times (R+N)}$$

In the production of N sheets by the discontinuous process, there will be N loading operations, N unloading operations and N consecutive fermentations. In this case, the total time to produce N sheets (indicated by $T_D$) will be:

$$T_D = N \times (TF) + N \times (TF)$$

And remembering that: $(TM) = R (TF)$, the result is that:

$$T_D = N \times (TF) (1+R)$$

The productivity of the discontinuous process $(P_D)$ will then be:

$$P_D = \frac{N}{T_D} = \frac{1}{(TF) \times (1+R)}$$

By dividing the value of $P_C$ by the value of $P_D$, we have:

$$\frac{Pc}{Pd} = (1 + R) \cdot \frac{N}{N + R}$$

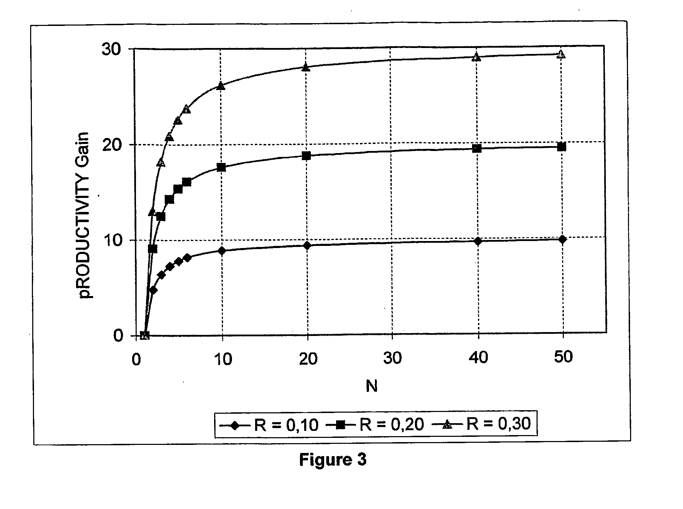

Knowing the values of N and R, the last equation allows us to calculate how many times the productivity of the continuous process is higher than the discontinuous one. Table 5 and Figure 3 show the results obtained for different values of N and R.

Table 5

| N | Increase in Productivity (%) | | |
|---|---|---|---|
| | R=0.10 | R=0.20 | R=0.30 |
| 1 | 0,0 | 0,0 | 0,0 |
| 2 | 4,8 | 9,1 | 13,0 |
| 3 | 6,4 | 12,5 | 18,2 |
| 4 | 7,3 | 14,3 | 20,9 |
| 5 | 7,8 | 15,4 | 22,6 |
| 6 | 8,2 | 16,1 | 23,8 |
| 10 | 8,9 | 17,6 | 26,2 |
| 20 | 9,4 | 18,8 | 28,1 |
| 40 | 9,7 | 19,4 | 29,0 |
| 50 | 9,8 | 19,5 | 29,2 |

The last equation shows that, no matter how high the value of N is, $P_C/P_D$ will never be higher than (1+R). Therefore, if R = 0.20 for example, i. e. the "dead time" of loading and unloading the tray is 20% of the fermentation time, the increase in productivity when substituting the discontinuous for the continuous process will be of 20% (see Table 5 and Figure 3).

**Claims**

1. Continuous fermentation process to produce bacterial cellulosic sheets in trays, **characterized by** the obtainment of multiple bacterial cellulosic sheets without the need to replace the fermentation medium with new fermentation medium in the tray, said process comprising the following steps:

   a) preparation of the fermentation medium; dissolution of medium components in water;
   b) sterilization of the medium: heating of medium to 115°C, keeping it at that temperature for 20 minutes;
   c) cooling of the medium until its temperature reaches 30°C;
   d) medium inoculation with a suspension of bacteriae;
   e) distribution of the medium in the trays;
   f) the trays are left to rest during fermentation;
   g) harvest of the sheets, draining them over the corresponding trays;
   h) repetition of the fermentation in the trays left to rest during fermentation and harvest of the sheets from the trays, draining them over the corresponding trays until, after taking and draining the sheet, the volume of medium in the tray is between 15% and 25% of the initial volume;
   i) when the volume of 15% to 25% of the initial volume is reached, a new fermentation medium is added to the tray in order to reach the initial volume again;

j) repetition of the fermentation in the trays left to rest during fermentation and harvest of the sheets from the trays, draining them over the corresponding trays until, after taking and draining the sheet, the volume of medium in the tray is between 15% and 25% of the initial volume when new fermentation medium is added and the cycle is re-started.

2. Process, according to claim 1, **characterized by** the fact that the inoculum is prepared from a pure culture of the bacteria or lyophilized bacteria.

3. Process, according to claim 1, **characterized by** the fact that the inoculum is prepared from the residual water medium existing in the tray in fermentation.

4. Process, according to claims 1 to 3, **characterized by** the fact that the volumes of medium used, the temperature and the time of incubation depend on the composition of the medium and the microorganism used.

5. Process, according to claim 1, **characterized by** the fact that the volume of inoculated medium to be put in each tray varies between 25 and 100% of the volume of the tray.

6. Process, according to claim 1, **characterized by** fact that the time of fermentation depends on the composition of the fermentation medium, strain and temperature.

7. Process, according to claims 1 and 6, **characterized by** the fact that the fermentation time varies from 24 to 456 hours for a cellulose pellicle grammage that varies from 6 to 230 $g/m^2$.

8. Process, according to claims 1 to 7, **characterized by** the fact that the mass of the sheet taken from the trays varies from 100 to 1100 g.

9. Process, according to claims 1 to 8, **characterized by** the fact that the cellulose existing in the sheet taken from the trays represents less than 20% of the mass of the sheet.

10. Process, according to claims 1 to 8, **characterized by** recovering part of the water medium that impregnates the sheet.

11. Process, according to claim 10, **characterized by** recovering from 200 to 600 ml of the volume of water medium for a mass of sheet that varies from 100 to 1100 g.

12. Process, according to claims 1, 10 and 11, **characterized by** the fact that the reduction of the volume of medium in the trays is as high as the mass of the sheet taken off.

13. Process, according to claims 10 and 11, **characterized by** the fact that the water medium serves as inoculum for other trays, being its volume equal to 10 to 20% of the volume of the fermentation medium.

14. Process, according to claims 1 to 13, **characterized by** the fact that the mass of the sheet formed is bigger when the sheet/air interface area and the fermentation time required to form the sheet are increased.

15. Process, according to claim 1, **characterized by** reducing the volume of effluents and the load of their pollutants.

16. Process, according to claim 1, **characterized by** reducing the cost by reducing operations and raw material.

17. Process, according to claim 1, **characterized by** improving productivity of the process in at least 15% over the discontinuous process.

**Patentansprüche**

1. Kontinuierliches Fermentationsverfahren zur Herstellung von bakteriellen Zellulosefilmen in Schalen, **gekennzeichnet durch** das Erhalten mehrerer bakterieller Zellulosefilme ohne die Notwendigkeit, das Fermentationsmedium **durch** neues Fermentationsmedium in der Schale ersetzen zu müssen, wobei das Verfahren die folgenden Schritte umfasst:

a) Herstellen des Fermentationsmediums: Auflösen der Mediumkomponenten in Wasser;
b) Sterilisieren des Mediums: Erwärmen des Mediums auf 115°C, Halten desselben bei dieser Temperatur für 20 Minuten;
c) Kühlen des Mediums, bis seine Temperatur 30°C erreicht;
d) Mediumbeimpfung mit einer Suspension von Bakterien;
e) Verteilen des Mediums in den Schalen;
f) die Schalen werden während der Fermentation ruhen gelassen;
g) Ernten der Filme und Abtropfenlassen derselben über den entsprechenden Schalen;
h) Wiederholen der Fermentation in den Schalen, die während der Fermentation ruhen gelassen werden, und Ernten der Filme von den Schalen, Abtropfenlassen derselben über den entsprechenden Schalen, bis nach dem Entnehmen und Abtropfenlassen des Films das Volumen des Mediums in der Schale zwischen 15% und 25% des Anfangsvolumens beträgt;
i) wenn das Volumen von 15% bis 25% des Anfangsvolumens erreicht ist, wird ein neues Fermentationsmedium der Schale zugegeben, um wieder das Anfangsvolumen zu erreichen;
j) Wiederholen der Fermentation in den Schalen, die während der Fermentation ruhen gelassen werden, und Ernten der Filme von den Schalen, Abtropfenlassen derselben über den entsprechenden Schalen, bis nach dem Entnehmen und Abtropfenlassen des Films das Volumen des Mediums in der Schale zwischen 15% und 25% des Anfangsvolumens beträgt, und dann neues Fermentationsmedium zugegeben wird und der Zyklus erneut gestartet wird.

2. Verfahren nach Anspruch 1, **gekennzeichnet durch** die Tatsache, dass das Impfgut aus einer reinen Kultur der Bakterien oder lyophilisierten Bakterien hergestellt wird.

3. Verfahren nach Anspruch 1, **gekennzeichnet durch** die Tatsache, dass das Impfgut aus dem restlichen Wassermedium hergestellt wird, das in der Schale in Fermentation vorhanden ist.

4. Verfahren nach den Ansprüchen 1 bis 3, **gekennzeichnet durch** die Tatsache, dass die Volumina von verwendetem Medium, die Temperatur und Zeit einer Inkubation von der Zusammensetzung des Mediums und dem verwendeten Mikroorganismus abhängen.

5. Verfahren nach Anspruch 1, **gekennzeichnet durch** die Tatsache, dass das Volumen des beimpften Mediums, das in jede Schale einzubringen ist, zwischen 25 und 100% des Volumens der Schale variiert.

6. Verfahren nach Anspruch 1, **gekennzeichnet durch** die Tatsache, dass die Zeit der Fermentation von der Zusammensetzung des Fermentationsmediums, dem Stamm und der Temperatur abhängt.

7. Verfahren nach den Ansprüchen 1 bis 6, **gekennzeichnet durch** die Tatsache, dass die Fermentationszeit von 24 bis 456 Stunden für ein Zellulosepellikel-Grammgewicht variiert, das von 6 bis 230 g/m$^2$ variiert.

8. Verfahren nach den Ansprüchen 1 bis 7, **gekennzeichnet durch** die Tatsache, dass die Masse des Films, der von den Schalen entnommen wird, von 100 bis 1100 g variiert.

9. Verfahren nach den Ansprüchen 1 bis 8, **gekennzeichnet durch** die Tatsache, dass die Zellulose, die in dem Film vorhanden ist, der von den Schalen entnommen wird, weniger als 20% der Masse des Films darstellt.

10. Verfahren nach den Ansprüchen 1 bis 8, **gekennzeichnet durch** das Wiedergewinnen eines Teils des Wassermediums, das den Film imprägniert.

11. Verfahren nach Anspruch 10, **gekennzeichnet durch** das Wiedergewinnen von 200 bis 600 ml des Volumens des Wassermediums für eine Filmmasse, die von 100 bis 1100 g variiert.

12. Verfahren nach den Ansprüchen 1, 10 und 11, **gekennzeichnet durch** die Tatsache, dass die Verringerung des Mediumvolumens in den Schalen so hoch ist wie die Masse des entnommenen Films.

13. Verfahren nach den Ansprüchen 10 und 11, **gekennzeichnet durch** die Tatsache, dass das Wassermedium als Impfgut für andere Schalen dient, wobei sein Volumen gleich 10 bis 20% des Volumens des Fermentationsmediums ist.

**14.** Verfahren nach den Ansprüchen 1 bis 13, **gekennzeichnet durch** die Tatsache, dass die Masse des gebildeten Films größer ist, wenn die Film/Luft-Grenzfläche und die Fermentationszeit, die zur Bildung des Films erforderlich ist, erhöht sind.

**15.** Verfahren nach Anspruch 1, **gekennzeichnet durch** ein Verringern des Volumens von Abwässern und ihrer Schadstoffbeladung.

**16.** Verfahren nach Anspruch 1, **gekennzeichnet durch** ein Verringern der Kosten durch Verringern der Arbeitsgänge und des Rohmaterials.

**17.** Verfahren nach Anspruch 1, **gekennzeichnet durch** ein Verbessern der Produktivität des Verfahrens um mindestens 15% gegenüber dem diskontinuierlichen Verfahren.

**Revendications**

**1.** Procédé de fermentation continu destiné à produire des feuilles cellulosiques bactériennes dans des bacs, **caractérisé par** l'obtention de multiples feuilles cellulosiques bactériennes sans nécessité de remplacement du milieu de fermentation par un nouveau milieu de fermentation dans le bac, ledit procédé comprenant les étapes suivantes :

a) la préparation du milieu de fermentation ; la dissolution des composants du milieu dans de l'eau ;
b) la stérilisation du milieu : le chauffage du milieu à 115°C, son maintien à cette température pendant 20 minutes ;
c) le refroidissement du milieu jusqu'à ce que sa température atteigne 30°C ;
d) l'inoculation du milieu avec une suspension de bactéries ;
e) la distribution du milieu dans les bacs ;
f) le fait de laisser les bacs reposer pendant la fermentation ;
g) la récolte des feuilles, leur drainage sur les bacs correspondants ;
h) la répétition de la fermentation dans les bacs que l'on laisse reposer durant la fermentation et de la récolte des feuilles des bacs, de leur drainage sur les bacs jusqu'à ce que, après avoir retiré et drainé la feuille, le volume de milieu dans les bacs soit de 15 % à 25 % du volume initial ;
i) l'ajout d'un nouveau milieu de fermentation lorsque le volume de 15 % à 25 % du volume initial est atteint, de manière à atteindre de nouveau le volume initial ;
j) la répétition de la fermentation dans les bacs que l'on laisse reposer durant la fermentation et de la récolte des feuilles des bacs, leur drainage sur les bacs correspondants jusqu'à ce que, après avoir retiré et drainé la feuille, le volume de milieu dans le bac soit de 15 % à 25 % du volume initial lorsque le nouveau milieu de fermentation est ajouté et le cycle est recommencé.

**2.** Procédé selon la revendication 1, **caractérisé par le fait que** l'inoculum est préparé à partir d'une culture pure de la bactérie ou de la bactérie lyophilisée.

**3.** Procédé selon la revendication 1, **caractérisé par le fait que** l'inoculum est préparé à partir du milieu aqueux résiduel existant dans le bac durant la fermentation.

**4.** Procédé selon les revendications 1 à 3, **caractérisé par le fait que** les volumes de milieu utilisés, la température et la durée d'incubation dépendent de la composition du milieu et du microorganisme utilisés.

**5.** Procédé selon la revendication 1, **caractérisé par le fait que** le volume de milieu inoculé à placer dans chaque bac varie de 25 à 100 % du volume du bac.

**6.** Procédé selon la revendication 1, **caractérisé par le fait que** la durée de fermentation dépend de la composition du milieu de fermentation, de la souche et de la température.

**7.** Procédé selon les revendications 1 et 6, **caractérisé par le fait que** la durée de fermentation varie de 24 à 456 heures pour un grammage de pellicule de cellulose qui varie de 6 à 230 g/m$^2$.

**8.** Procédé selon les revendications 1 à 7, **caractérisé par le fait que** la masse de la feuille retirée dans les bacs varie de 100 à 1100 g.

9. Procédé selon les revendications 1 à 8, **caractérisé par le fait que** la cellulose existant dans la feuille retirée des bacs représente moins de 20 % en masse de la feuille.

10. Procédé selon l'une quelconque des revendications 1 à 8, **caractérisé par** une partie de récupération du milieu aqueux qui imprègne la feuille.

11. Procédé selon la revendication 10, **caractérisé par** la récupération de 200 à 600 mL du volume du milieu aqueux pour une masse de feuille qui varie entre 100 et 1100 g.

12. Procédé selon les revendications 1, 10 et 11, **caractérisé par le fait que** la réduction du volume du milieu dans les bacs est aussi élevée que la masse de la feuille retirée.

13. Procédé selon les revendications 10 et 11, **caractérisé par le fait que** le milieu aqueux sert d'inoculum pour les autres bacs, son volume étant égal à 10 à 20 % du volume du milieu de fermentation.

14. Procédé selon les revendications 1 à 13, **caractérisé par le fait que** la masse de la feuille formée est plus grande lorsque l'interface feuille/air et la durée de fermentation requises pour former la feuille sont accrues.

15. Procédé selon la revendication 1, **caractérisé par** la réduction du volume d'effluents et de la charge de leurs produits polluants.

16. Procédé selon la revendication 1, **caractérisé par** la réduction du coût par la réduction des opérations et de la matière première.

17. Procédé selon la revendication 1, **caractérisé par** l'amélioration de la productivité du procédé d'au moins 15 % par rapport au procédé discontinu.

**Figure 1**

**Figure 2**

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- BR PI9204232
- BR 8404937
- BR 1984
- US 4912049 A
- US 1990 A
- BR PI02054990
- WO 2004050986 A

### Non-patent literature cited in the description

- *Biochemical Journal,* 1956, vol. 58, 345-352
- *Brazilian Journal of Chemical Engineering,* 1996, vol. 13, 47-50
- *Carbohydrate Polymers,* 1999, vol. 40, 137-143
- *Journal of Bioscience and Bioengineering,* 1999, vol. 88, 183-188
- *Biotechnology and Bioengineering,* 2000, vol. 68, 345-372
- *Biotechnology and Applied Biochemistry,* 2002, vol. 35, 125-132
- **Brown, A.J.** *J.Chem.Soc.,* 1900, vol. 49, 432
- **Tarr ; Hibbert.** *Can. J. Res.,* 1931, vol. 4, 373
- **Khouvine, Y.** *Bull. Soc. Chem.,* 1936, vol. 18, 1325
- **Hestrin, S. ; Aschner, M. ; Mager, J.** Synthesis of cellulose by resting cells of Acetobacter xylinum. *Nature. London,* 1947, vol. 159, 64
- **Kaushall, R. ; Walker, T.K. ; Drummond, D. G.** Observations on the formation and structure of bacterial cellulose. *Biochem. J.,* 1951, vol. 50, 128
- **Colvin, J. R. ; Chènè, L. ; Sowden, L. C. ; Takai, M.** Purification and properties of a soluble polymer of glucose from cultures of Acetobacter xylinum. *Canadium Journal of Biochemistry,* 1977, vol. 55, 1057-1063
- The biosynthesis of cellulose. **Colvin, J. R.** Biochemistry of Plants, vol. 3, Carbohydrates; Structure and Function. Academic Press, 1980, vol. 3, 543-570
- **Leppard, G.G. ; Sowden, L.C. ; Colvin J. R.** *Science,* 1975, vol. 189, 1094-1095
- **Borzani ; Souza.** *Biotechnology Letters,* 1995, vol. 17, 1271-1272